# EUROPEAN PATENT SPECIFICATION

(11) **EP 1 445 266 B1**
(45) Date of publication and mention of the grant of the patent: **03.05.2006**
(21) Application number: 04008994.8
(22) Date of filing: 20.05.1999
(51) Int. Cl.: C08F 220/18, C08F 220/30, G03F 7/039

(54) **Photoresist copolymer**
Photoresist copolymer.
Copolymère photosensible.

(30) Priority: 25.05.1998 JP 14353698; 28.08.1998 JP 24406798
(43) Date of publication of application: 11.08.2004
(62) Divisional of application: 99953334.2
(73) Proprietor: DAICEL CHEMICAL INDUSTRIES, LTD., Sakai-shi, Osaka 590-8501 (JP)
(72) Inventor: Nakano, Tatsuya, Himeji-shi Hyogo 670-0094 (JP)
(74) Representative: Grünecker, Kinkeldey, Stockmair & Schwanhäusser Anwaltssozietät

(56) References cited:
- JP-A- 7 196 743
- JP-A- 7 234 511
- JP-A- 8 012 626
- JP-A- 8 082 925
- JP-A- 10 003 169
- JP-A- 10 083 076
- JP-A- 10 312 060
- JP-A- 11 109 632

## Description

The present invention relates to a photoresist copolymer, a photoresist resin composition comprising same, and a method of forming a pattern in which said photoresist resin composition is used. The photoresist resin composition is suitable for the formation of a pattern (e.g. minute (fine) processing of a semiconductor) using ultraviolet rays or far-ultraviolet rays (inclusive of excimer laser beams).

The semiconductor integrated circuit is fabricated by a lithographic process which comprises forming a resist thin-layer on a substrate, then forming a latent image pattern by imagewise exposure to light, developing the latent image to form a resist pattern, dry-etching the substrate using the resist pattern as a mask, and removing the resist to provide a designed pattern.

As the resist for semiconductor manufacture, a photosensitive resin composition containing an alkali-soluble novolac resin and a diazonaphtoquinone derivative is known. This resin composition has been used as a positive-acting resist by taking advantage of the phenomenon that on exposure to light the diazonaphthoquinone group is decomposed to give a carboxyl group, whereby the composition which is initially alkaliinsoluble is rendered alkali-soluble. There also is known a negative-acting resist, that is a resist which becomes insoluble on exposure to light through a photo-crosslinking reaction in the presence of an azide compound or a photopolymerization reaction in the presence of a photopolymerization initiator.

Meanwhile, in the lithographic technology, the demand for a finer line pattern definition has caused a shift from ultraviolet rays, such as g-line and i-line, to rays of shorter wavelengths, such as far-ultraviolet rays, vacuum ultraviolet rays, excimer laser beams, an electron beam and X-rays.

However, because the resins used contain an aromatic ring, those resists may at times be opaque to light at wavelengths shorter than 200 nm and are not suited (inactive) as compositions for use with an ArF excimer laser which has a wavelength of 193 nm.

As a photoresist suitable for short-wavelength exposure light sources (e.g. ArF excimer laser), Japanese Patent Application Laid-Open No, 73173/1997 discloses a resist material comprising a polymer having a structural unit protected by an alicyclic hydrocarbon group, such as adamantane or norbornane, which is cleaved (eliminated) by an acid to render the material alkali-soluble in combination with an acid precursor. This literature mentions, as the polymer, (1) a copolymer of 2-methyl-2-adamantyl (meth)acrylate and (2) a copolymer of 2-(1-adamantyl)propyl (meth)acrylate, among others. The above polymer having no double bond within its ring structure is transparent (active) to the ArF excimer laser beam and, in semiconductor fine processing, the resistance to plasma gas dry-etching is enhanced.

However, when the above resist comprising the polymer and the acid precursor is used to form a pattern, the tendency toward formation of cracks and peeling of the pattern is increased as the pattern line becomes finer so that it is sometimes impossible to form a pattern of fine line definition.

The present invention, therefore, has the object to provide a photoresist copolymer derived from an acid-responsive compound having an adamantane skeleton and capable of being alkali-soluble on exposure to light, thus being useful for the formation of fine-line patterns.

It is another object of the present invention to provide a photoresist copolymer which is high in sensitivity and etching resistance (particularly resistance to dry etching) and instrumental in forming a fine-line resist pattern with good reproducibility and high precision.

It is a still another object of the present invention to provide a photoresist resin composition showing high adhesion to a substrate and useful for forming a fine-line resist pattern with high precision and high reproducibility.

The inventors of the present invention did intensive investigations for accomplishing the above objects and found that when a copolymer comprising a specific acid-responsive compound unit having an adamantane group and a specific structure is used in combination with a photoactive acid precursor, the adamantane group is stably and efficiently eliminated from the copolymer by the acid formed from the acid precursor on exposure to light to thereby enable water or alkali development. The present invention has been developed on the basis of the above finding.

The present invention provides a photoresist copolymer comprising
a monomer unit corresponding to a monomer represented by the following formula (11a) or (12a): 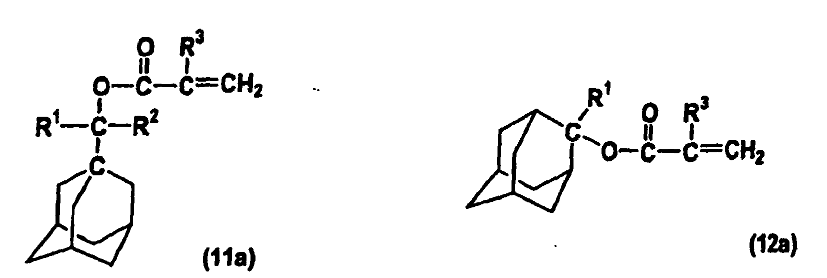 wherein R¹ and R² are the same or different and each represents a C₁₋₄alkyl group, and R³ represents a hydrogen atom or methyl group; and
a lactone ring-containing monomer unit.

The present invention further provides a photoresist resin composition comprising the above photoresist copolymer and a photoactive acid precursor.

Moveover, the invention is directed to a method of forming a pattern which comprises the steps of:
forming a layer comprising the above photoresist resin composition on a substrate;
subjecting said layer to pattern exposure; and
developing the exposed coating layer to form a pattern.

Preferred embodiments of the invention are set forth in the sub-claims.

The adamantane-based monomers used in the present invention are represented by formulae (11a) and (12a): 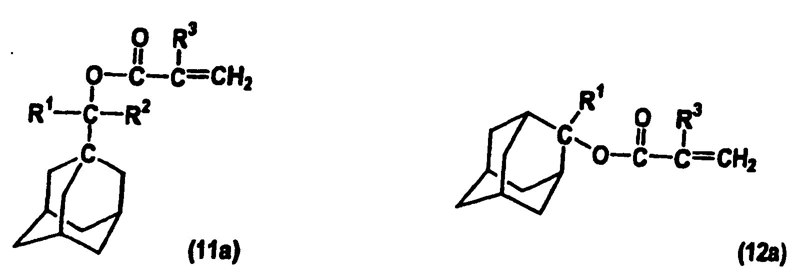 The alkyl groups represented by R¹ and R² each includes straight-chain or branched-chain C₁₋₄ alkyl groups , such as methyl, ethyl, propyl, isopropyl, butyl, isobutyl, s-butyl, and t-butyl.

The branched-chain alkyl group may be an alkyl group containing a tertiary carbon atom. The branched-chain alkyl group having a tertiary carbon atom includes alkyl groups such as isopropyl, isobutyl, 1-methylethyl, and 1-ethylethyl. The preferred branched-chain alkyl group R¹ includes alkyl groups having a methine carbon atom in the α-position. The alkyl group designated by R¹ is preferably a branched-chain alkyl group containing a tertiary carbon atom.

The cycloalkyl group includes cyclopropyl, and cyclobutyl.

R³ represents a hydrogen atom or a methyl group, forming an acryloyl or methacryloyl group.

R¹ and R² may, jointly and together with the adjacent carbon atom, form an alicyclic hydrocarbon ring. This alicyclic hydrocarbon ring includes hydrocarbon rings corresponding to the cycloalkyl group.

The acid-responsive (acid-sensitive) compounds (11a) and (12a) used in the present invention can be prepared, for example, in accordance with the following reaction schemes. 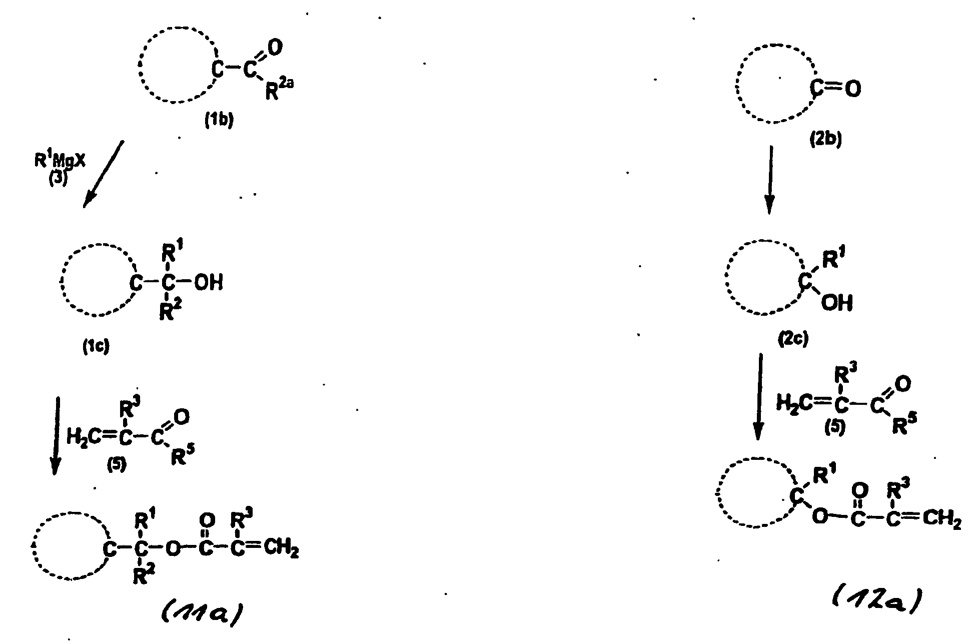 wherein 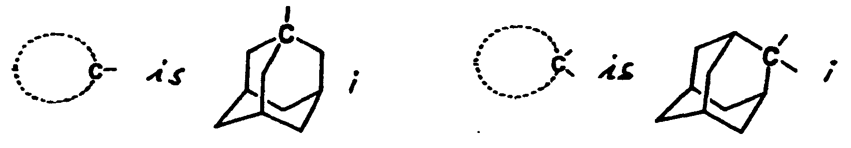 X represents a halogen atom; R^{2a} means the same as R² or represents a halogen atom; R⁵ represents a halogen atom or a hydroxyl group, an alkoxy group, an alkenyloxy group or an alkynyloxy group; R¹, R² and R³ are each as defined hereinbefore.

The halogen atom typically includes chlorine, bromine and iodine, and the alkoxy group includes C₁₋₁₀ alkoxy groups (e.g. methoxy, ethoxy, t-butoxy). The alkenyloxy group includes C₂₋₁₀ alkenyloxy groups (e.g. vinyloxy, allyloxy, 1-propenyloxy, isopropenyloxy, 1-butenyloxy, 2-butenyloxy, 3-butenyloxy, 2-pentenyloxy), and the alkynyloxy group includes C₂₋₁₀ alkynyloxy groups (e.g. ethynyloxy, propynyloxy).

Referring to the above reaction schemes, the carbonyl compound (1b) includes, to mention typical examples, adamantyl-1-C₂₋₆ alkanones. e.g. adamantan-1-yl-ethan-1-one, adamantan-1-yl-propan-1-one, adamantan-1-yl-butan-1-one, methyladamantan-1-yl-ethan-1-one).

The carbonyl compound (1b) in which R^{2a} is a halogen atom, i.e. the acid halide, includes adamantane-1-carbonyl halldes).

The carbonyl compound (2b) includes adamantanone.

In the above reaction schemes, the reaction of the carbonyl compound (1b) or (2b) with the reagent R¹MgX (3) can be carried out according to the conventional Grignard reaction. The amount of the Grignard reagent R¹MgX (3) relative to 1 mol of the carbonyl compound (1b) or (2b) may for example be 0.8 to 3 mols (e.g. 1 to 2.5 mols), preferably 1 to 2 mols, more preferably 1 to 1.5 mols. When the carbonyl compound (1b) to be used has a halogen atom for R^{2a}, the compound (1c) wherein R² is the same as R¹ can be produced by reacting 2 mols of the Grignard reagent R¹MgX (3) relative to 1 mol of the carbonyl compound (1b).

This reaction can be conducted in an inert solvent to the reaction, such as hydrocarbons (hexane, cyclohexane), ethers (dimethyl ether, diethyl ether, tetrahydrofuran), to mention just a few preferred solvents. The reaction temperature can be suitably selected from the range of, for example, 0 to 100°C, preferably 10 to 50°C.

The hydroxy compound (1c) or (2c) formed by the reaction, optionally isolated, is subjected to the esterification reaction using (meth)acrylic acid or a derivative thereof (5) to give the acid-responsive compound (11a) or (12a).

The (meth) acrylic acid or its derivative (5), mentioned above, includes (meth)acrylic acid, (meth)acrylic anhydride, and reactive derivatives having a leaving group [e.g. acid halides ((meth)acryloyl chloride, (meth)acryloyl bromlde), (meth) acrylic acid alkyl esters such as C₁₋₆ alkyl (meth)acrylates (e.g. methyl (meth)acrylate, ethyl (meth)acrylate, propyl (moth)acrylate, butyl (meth)acrylate, isobutyl (meth)acrylate, t-butyl (meth)acrylate), (meth)acrylic acid alkenyl esters (e.g. C₂₋₁₀ alkenyl (meth)acrylates such as vinyl (meth)acrylate, allyl (meth)acrylate, 1-propenyl (meth)acrylate, isopropenyl (meth)acrylate, 1-butenyl (meth)acrylate, 2-butenyl (meth)acrylate, 3-butenyl (meth)acrylate, 2-pentenyl (meth)acrylate), and alkynyl esters of (meth) acrylic acid (e.g. C₂₋₁₀ alkynyl (meth)acrylates such as ethynyl (meth)acrylate, propynyl (math) acrylate)].

The preferred compound (5) includes (meth) acrylic acid, (meth)acryloyl halides, C₁₋₆ lower alkyl esters of (meth)acrylic acid, C₂₋₆ alkenyl esters of (meth) acrylic acid, and C₂₋₆ alkynyl esters of (meth)acrylic acid. Particularly with a (meth)acryloyl halide or a C₂₋₆ alkenyl (meth)acrylate, the corresponding acid-responsive compound can be obtained with high selectivity and in high yield through a leaving group exchange reaction while side reactions such as addition polymerization are inhibited.

The above esterification reaction can be carried out by a conventional manner, for example in the presence of a suitable catalyst (an acid catalyst). When a (meth)acryloyl halide is used, there are cases in which the acid-responsive compound is contaminated with the halogen component. Therefore, this esterification reaction is preferably effected by the esterification reaction using (meth)acrylic acid or the transesterification reaction. The esterification reaction and transesterification reaction can be conducted using the conventional esterification catalyst (for example a non-halogen series inorganic acid such as sulfuric acid, hydrochloric acid, a sulfonic acid such as p-toluenesulfonic acid, a protonic acid such as acidic ion exchange resin, a Lewis acid such as boron trifluoride, an enzyme) and a transesterification catalyst (for example, the esterification catalysts, alkali metal alkoxide such as sodium alkoxides, aluminum alkoxides, titanic acid esters).

To enhance the reaction efficiency and obtain the objective acid-responsive compound in high yield, the esterification reaction (inclusive of leaving group exchange reactions such as transesterification) between the hydroxy compound (1c) or (2c) and (meth)acrylic acid or a derivative thereof (5) is conducted with advantage in the presence of a catalyst comprised of a compound of a Group 3 element of Periodic Table of the Elements. In the reaction utilizing such a catalyst, the formation of the amine hydrochloride can be inhibited and, when a C₁₋₄ lower alkyl ester or C₂₋₄ alkenyl ester of (meth)acrylic acid is used, the objective compound can be protected against contamination with the halogen component. Moreover, because a low-boiling compound (e.g. the above ester) can be used as the (meth) acrylic acid or derivative (5), the treatment after the reaction is easy and the isolation yield can be dramatically increased.

Referring to the catalyst comprised of a compound of Group 3 element, the Group 3 element includes rare earth elements, e.g. scandium, yttrium, lanthanide series elements (lanthanum, cerium, praseodymium, neodymium, promethium, samarium, europium, gadolinium, terbium, dysprosium, holmium, erbium, thulium, ytterbium, lutetium)], and actinoid series elements (e.g. actinium). The preferred Group 3 element includes rare earth elements, such as scandium, yttrium, and lanthanoid series elements (samarium, gadolinium, ytterbium).

The valence of the Group 3 element is not particularly restricted but is often bivalent to tetravalent, particularly bivalent or trivalent. The compound of Group 3 element, mentioned above, is not particularly restricted only if it has the necessary catalyst activity, and may be an elemental metal or a compound or complex of the metal with an inorganic compound (e.g. a halide, oxide, double oxide, phosphorus compound or nitrogen compound) or an organic compound (e. g. an organic acid) . In many instances, it is the hydroxide or oxo-acid salt, organic acid salt, inorganic acid salt or halide containing the metal element or a coordination compound (complex) containing the metal element. The complex may be a π complex such as a metallocens compound. Furthermore, the compound of Group 3 element may be a double salt compound (complex metal compound) with another metal. Those catalysts can be used each alone or in a combination of two or more species.

The catalyst component is now described in further detail taking a samarium compound as an example, it being, however, to be understood that the compounds of other Group 3 elements which correspond to the samarium compound can be used likewise with success.

The hydroxide includes samarium (II) hydroxide and samarium (III) hydroxide, for instance, and the metal oxide includes samarium (II) oxide and samarium (III) oxide, for instance.

The organic acid salt includes salts with such organic acids as organic carboxylic acids (monocarboxylic acids, polycarboxylic acids), hydroxycarboxylic acids, thiocyanic acid or sulfonic acids (alkylsufonic acids, benzenesufonic acid, arylsulfonic acids). The inorganic acid salt includes the nitrate, sulfate, phosphate, carbonate and perchlorate. The organic acid salt and inorganic acid salt, mentioned above, include samarium acetate, samarium trichloroacetate, samarium trifluoroacetate, samarium trifluoromethanesulfonate (i.e. samarium triflate), samarium nitrate, samarium sulfate, samarium phosphate and samarium carbonate.

The halide may for example be the fluoride, chloride, bromide or iodide.

The ligand forming the complex includes OH (hydroxo), alkoxy, acyl, alkoxycarbonyl, acetylacetonato, cyclopentadienyl, C₁₋₄alkyl-substituted cyclopentadienyl (e.g. C₁₋₂ alkyl-substituted cyclopentadienyl groups such as pentamethylcyclopentadienyl), dicyclopentadienyl, C₁₋₄ alkyl-substituted dicyclopentadienyl (e.g. C₁₋₂ alkyl-substituted dicyclopentadienyl such as pentamethyldicyclopentadienyl), halogen, CO, CN, oxygen, H₂O (aqua); phosphorus compounds such as phosphines, nitrogen-containing compounds such as NH₃ (ammine), NO, NO₂ (nitro), NO₃ (nitrato), ethylenediamine, diethylenetriamine, pyridine, phenanthroline. Referring to the complexes or complex salts, one or more similar or dissimilar ligands may be coordinated.

Among the complexes, samarocene complexes include diacetylacetonatosamarium (II), triacetylacetonatosamarium (III), dicyclopentadienylsamarium (II), tricyclopentadienylsamarium (III), dipentamethylcyclopentadienylsamarium (II) and tripentamethylcyclopentadienylsamarium (III).

When the compound of Group 3 element [the bivalent samarocene complex having the pentamethylcyclopentadienyl ligands which are highly electron-donative [(C₅Me₅)₂Sm; (PMSm)] or any of samarium compounds such as samarium halides, alkoxide, hydroxide] is used as the catalyst, esterification proceeds with a higher efficiency and with side reactions inhibited as compared with the reaction in the presence of a Lewis acid catalyst or a protonic acid catalyst even in esterification reactions which are handicapped as equilibrium reactions. Thus, this catalyst is useful for producing the acid-responsive compounds (11a) and (12a) by a leaving group exchange reaction such as transesterification.

The catalyst comprised of a compound of Group 3 element may be a homogeneous system or a heterogeneous system. Moreover, the catalyst may be an solid catalyst comprising the compound of Group 3 element supported on a support or carrier. The support is usually a porous support such as activated carbon, zeolite, silica, silica-alumina, or bentonite. The amount of the supported catalyst component is 0.1 to 50 parts by weight, preferably 0.5 to 30 parts by weight, more preferably 1 to 20 parts by weight relative to 100 parts by weight of the support.

The amount of the catalyst (e.g. the catalyst comprised of the compound of Group 3 element) can be liberally selected from a broad range, for example the range of 0.1 mol % to 1 equivalent, preferably 0.5 to 50 mol %, more preferably 1 to 25 mol. (e.g. 5 to 20 mol %), based on the hydroxy compound (1c) or (2c).

The esterification reaction mentioned above (particularly the reaction using the compound of Group 3 element as the catalyst) may be conducted in the presence of an oxime. The oxime may be whichever of an aldoxime or a ketoxime and includes aliphatic oximes such as 2-hexanone oxime, alicyclic oximes such as cyclohexanone oxime, and aromatic oximes such as acetophenone oxime, benzophenone oxime, and benzyl dioxime.

The amount of the oxime can be selected from a broad range, for example the range of 0.1 mol % to 1 equivalent, preferably 1 to 50 mol %, more preferably 5 to 40 mol % (e.g. 5 to 30 mol %), based on the hydroxy compound (1c) or (2c).

The ratio of (meth)acrylic acid or its derivative (5) to hydroxy compound (1c) or (2c) is 0.5 to 5 mols, preferably 0.8 to 5 mols, more preferably not less than 1 mol (e.g. 1 to 3 mols, particularly 1 to 1.5 mols) of (meth)acrylic acid or derivative (5) per equivalent of hydroxy compound (1c) or (2c) (that is the weight of the hydroxy compound per hydroxyl group). Since the esterification reaction is an equilibrium-controlled reaction, it is more advantageous to use a larger proportion of (moth) acrylic acid or derivative thereof (5) for accelerating the reaction but because of the usually high catalyst activity of the compound of Group 3 element, (meth)acrylic acid or derivative thereof (5) need not be used in a large excess. Particularly, in the reaction involving a combination of reactants which is very unfavorable from the standpoint of reaction equilibrium, the use of the alkenyl ester having a vinyl leaving group (e.g. vinyl ester) as the (meth)acrylic acid or derivative thereof (5) rather leads, in many instances, to an early completion of reaction and a better result even if the compound (1c) is used in a proportion of only 1 mol or less (e.g. 0.4 to 1 mol, particularly 0.5 to 1 mol) per equivalent of the leaving group of hydroxy compound (1c) or (2c).

In the process using the catalyst, the heat of reaction is not so high as in the process using an acid halide such as (meth)acryloyl chloride so that the reaction can be smoothly conduced in a small amount of solvent and the end product can be obtained in a higher yield.

The esterification reaction mentioned above can be carried out in the presence or absence of a solvent inert to the reaction. The reaction solvent which can be used includes aliphatic hydrocarbons, alicyclic hydrocarbons, aromatic hydrocarbons, polar aprotic solvents such as ketones, ethers, amides, N-methylpyrrolidone, nitriles, and mixed solvents thereof. As the reaction solvent, (meth)acrylic acid or derivative thereof (5) may be used.

When a highly hydrophilic species of hydroxy compound (1c) or (2c) is used, the solvent may be a hydrophilic solvent (ketones such as acetone, methyl ethyl ketone, ethers such as dioxane, diethyl ether, tetrahydrofuran, and other polar aprotic solvents), or a mixed solvent of a hydrophilic solvent and a hydrophobic solvent (an aliphatic, alicyclic or aromatic hydrocarbon).

Since the above reaction is an equilibrium-controlled reaction, it is advantageous to remove the reaction-interfering components such as the cleaved (eliminated) component from the reaction system for accelerating the reaction. For removal of the eliminated component, it is advantageous to use a high-boiling solvent (for example an organic solvent boiling at 50 to 120°C, particularly 60 to 115°C) or an azeotropic solvent (for example the hydrocarbons).

The esterification reaction temperature can be selected from the range of, for example, 0 to 150°C, preferably 25 to 120°C. When the catalyst comprised of the compound of Group 3 element is used, the acid-responsive compound forms with high efficiency even under mild conditions. Thus, the reaction temperature may for example be 0 to 150°C, preferably 10 to 100°C, more preferably 20 to 80°C. Particularly when the alkenyl ester, for instance, is used as the (meth)acrylic acid or derivative (5), the reaction can be smoothly carried through even under mild conditions, namely at 20 to 50°C. The reaction can be conduced at atmospheric pressure, under applied pressure or at elevated pressure. Moreover, the reaction can be carried out batchwise, semi-batchwise or continuously in the conventional manner.

After completion of the reaction, the acid-responsive compound (11a) or (12a) can be easily isolated and purified by such separatory means as filtration, concentration, distillation, extraction, crystallization, recrystallization, column chromatography, as applied independently or in combination.

### [Photoresist resin composition]

The photoresist resin composition of this invention is characterized in that at least a copolymer containing the unit of formula (11a) or (12a) (a unit having an adamantane skeleton) and a photoactive acid precursor (photoactive acid generator) are used in combination and that the copolymer is solubilized by irradiation with light. Thus, probably because the ester bond (linkage) which is adjacent to the bulky, hydrophobic adamantane ring is stably eliminated with high efficiency by the acid produced by the irradiation, a fine-line resist pattern can be formed with high accuracy and reproducibility while the high sensitivity and high etching resistance are maintained.

Furthermore, because a copolymer containing the acid-responsive compound (11a) or (12a) as a repeating unit is highly soluble in a organic solvent for photoresist use, the precipitation of the polymer can be prevented and the stability of the photoresist solution be improved. In addition, because of the high adhesion of the solution to the substrate, resist patterns can be obtained with high accuracy. Moreover, the cleaning and removing by a development procedure such as dry etching after pattern exposure can be neat and thorough so that the sensitivity of pattern formation is high.

Since the monomer contains an adamantane ring, the dry-etching resistance is high and the degree of developer-induced swelling is low so that the circuit pattern can be formed with good accuracy.

The lactone ring-containing monomers include (meth)acrylic monomers of the following formula: 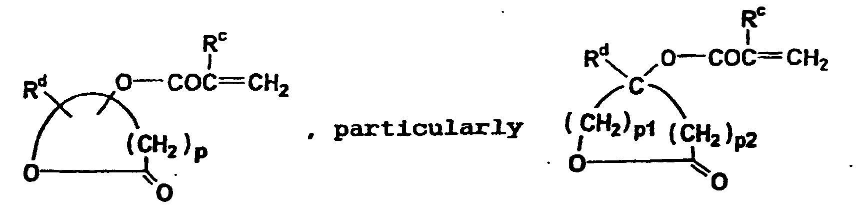 wherein R^{c} represents a hydrogen atom or a methyl group; R^{d} represents a hydrogen atom or a C₁₋₄ alkyl group; p represents an integer of 2 to 15; p1 and p2 each represents an integer of 0 to 8; p1+p2 = 1 to 14
and the allyl monomers corresponding to the above monomers.

The C₁₋₄ alkyl group mentioned for R^{d} includes methyl, ethyl, propyl, isopropyl, butyl, isobutyl and t-butyl. R^{d} is usually a hydrogen atom or a methyl group. The integer p is usually 3 to 10, particularly 3 to 6. Moreover, p1 and p2 each is usually an integer of 0 to 8, and p1+p2 = 2 to 9 (preferably 2 to 5). The numbers and substitution positions of the (meth)acryloyloxy group, allyloxy group and Rd are not particularly restricted; thus, they may be located in suitable positions on the lactone ring.

Those copolymerizable monomers may have a variety of substituents (e.g. polar groups such as oxo, hydroxyl, alkoxy, carboxyl, alkoxycarbonyl, cycloalkyloxycarbonyl, aryloxycarbonyl, aralkyloxycarbonyl, hydroxymethyl, carbamoyl, N-substituted carbamoyl, or nitro). Those copolymerizable monomers can be used each alone or in a combination of two or more species.

The proportion of the acid-responsive compound (11a), (12a), in the copolymer may for example be 15 to 90 weight %, preferably 30 to 75 weight %, more preferably 35 to 70 weight %.

The photoactive acid precursor which can be used includes the conventional compounds which, when exposed to light, form acids (protonic acids or Lewis acids) with high efficiency, for example diazonium salts, iodonium salts, sulfonium salts, oxathiazole derivatives, s-triazine derivatives, imide compounds, oxime sulfonates, diazonaphthoquinone, sulfonic esters [1-phenyl-1-(4-methylphenyl)sulfonyloxy-1-benzoylmethane, 1,2,3-trisulfonyloxymethylbenzene, 1,3-dinitro-2-(4-phenylsulfonyloxymethyl)benzene, 1-phenyl-1-(4-methylphenyl)sulfonyloxymethyl-1-hydroxy-1-benzoylmethane, disulfone derivatives (diphenyl disulfone), benzoin tosylate or Lewis acids (triphenylsulfonium hexafluoroantimonate ((Ph)₃S⁺SbF₆⁻), triphenylsulfonium hexafluorophosphate ((Ph)₃S⁺PF₆⁻), triphenylsulfonium methanesulfonyl ((Ph)₃S⁺CH₃SO₃⁻), diphenyliodonium hexafluorophosphate). In the above description, Ph represents a phenyl group.

Those photoactive acid precursors can be used each alone or in a combination of two or more species.

The amount of the photoactive acid precursor can be selected according to the strength of the acid that will be produced by irradiation and the amount of the acid-responsive compound, among other conditions, for example from the range of 0.1 to 30 parts by weight, preferably 1 to 25 parts by weight, more preferably 2 to 20 parts by weight, relative to 100 parts by weight of the copolymer.

The photoresist resin composition may contain an alkali-soluble component such as alkali-soluble resin (novolac resin, phenolic resin, carboxyl-containing resin), a coloring agent (dye), an organic solvent. The organic solvent includes hydrocarbons, halogenated hydrocarbons, alcohols, esters, ketones, ethers, cellosolves (methylcellosolve, ethylcellosolve, butylcellosolve), carbitols, glycol ether esters (mono- or polyalkylene glycol monoalkyl ether esters, cellosolve acetates such as ethylcellosolve acetate, propylene glycol monomethyl ether acetate, propylene glycol monoethyl ether acetate) and mixed solvents thereof.

Further, the photoresist resin composition may be freed of contaminants by the conventional separation and purification procedure such as the use of a filter.

The photoresist resin composition of this invention can be prepared by mixing the copolymer and the photoactive acid precursor. As this photoresist resin composition is coated on a substrate or base and dried and the resulting layer (resist layer or resist film) on the substrate is imagewise exposed to light and developed, a pattern corresponding to the exposure pattern can be formed on the substrate, with high sensitivity to exposure light and high pattern resolution. Usually, the film is exposed to light through a suitable mask to form a latent image, which can then be developed to provide a fine-line pattern with high accuracy.

The substrate or base can be selected according to the intended use of the photoresist resin composition and may for example be a silicon wafer or a metal, plastic, glass, ceramic or other substrate. The coating with the photoresist resin composition can be effected by the conventional procedure suited to each application, for example by the spin coating technique or the roll coating technique. The thickness of coating layer comprising the photoresist resin composition can be judiciously selected from the range of, for example, 0.1 to 20 µm.

For the exposure to light, light beams of various wavelengths, e.g. ultraviolet rays and X-rays, can be employed. For the exposure of resists for semiconductor manufacturing, g-line, i-line, and excimer laser (e.g. XeCl, KrF, KrCl, ArF. ArCl) beams can be utilized.

The exposure beam energy can be selected from the range of, for example, 1 to 1000 mJ/cm ², preferably 10 to 500 mJ/cm².

On irradiation, an acid is generated from the acid precursor and the group containing the adamantane ring (usually the alcohol containing the adamantane ring) is eliminated by the generated acid and a carboxyl group contributory to solubilization is generated. Therefore, the desired pattern can be formed using an aqueous developer or an alkali developer. Particularly because the photoresist resin composition of the present invention has the adamantane ring, it is highly resistant to etching (dry etching in particular) so that an elaborate circuit pattern can be formed with high accuracy. Incidentally, the cleavage of the group containing the adamandane ring may be promoted by exposure and post-exposure baking (PEB).

The present invention can be embodied in various applications, such as circuit-forming materials (resists for semiconductor manufacturing, printed circuit boards) and image-forming materials (printing plates, relief images).

### INDUSTRIAL APPLICABILITY

The acid-responsive compound used in the present invention has an alicyclic hydrocarbon group (an adamantane skeleton) and becomes alkali-soluble upon exposure to light, with the result that it finds application as a photoresist in the formation of fine-line patterns. Furthermore, because it is high in sensitivity and etching resistance (dry etching resistance in particular), fine-line patterns can be formed with good reproducibility and high accuracy. In addition, it contributes to improved adhesion to the substrate and improved stability of the resist solution, thus insuring the formation of fine-line patterns with high accuracy and good reproducibility.

### EXAMPLES

The following examples show the preparation of the adamantane-based monomers.

### Example 1

### (1) Hydroxylation

To a solution of adamantan-1-yl-ethan-1-one (1 mol) in absolute tetrahydrofuran was added a solution of Isopropylmagnesium iodide (iso-C₃H₇MgI) (1.2 mols) in absolute diethyl ether dropwise, and the mixture was stirred at 10°C for 6 hours to provide 1-(1-hydroxy-1,2-dimethylpropyl) adamantane.

### (2) Esterification

A mixture of 1-(1-hydroxy-1.2-dimethylpropyl)adamantane obtained above (1.00 mmol), samarium iodide (SmI₂) (0.10 mmol), isopropenyl acrylate (1.1 mmols) and dioxane (2 mmols) was stirred at 50°C for 6 hours. Analysis by gas chromatography revealed the formation of 1-(1-acryloyloxy-1,2-dimethylpropyl)adamantane of the following formula (yield 90%) in the reaction mixture. Mass spectrum: [M] 276, 261, 218, 147, 135.

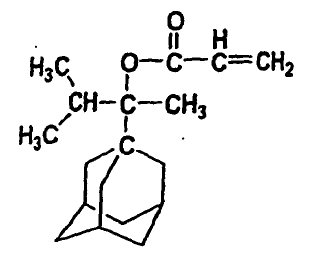

### Example 2

### (1) Hydroxylation

Using adamantanone in lieu of adamantan-1-yl-ethan-1-one, Step (1) of Example 1 was otherwise repeated to provide 2-isopropyl-2-hydroxyadamantane at the conversion rate of 76% (yield 61%).

### (2) Esterification

Using 2-isopropyl-2-hydroxyadamantane in lieu of 1-(1-hydroxy-1,2-dimethylpropyl)adamantane. Step (2) of Example 1 was otherwise repeated to provide the compound of the following formula, namely 2-isopropyl-2-acryloyloxyadamantane (yield 78%).
Mass spectrum of the compound: [M] 248, 233, 218, 205, 183, 139.

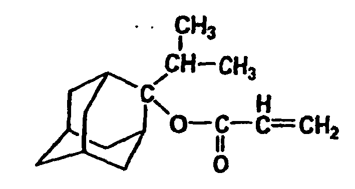

## Claims

1. A photoresist copolymer comprising
a monomer unit corresponding to a monomer represented by the following formula (11a) or (12a): 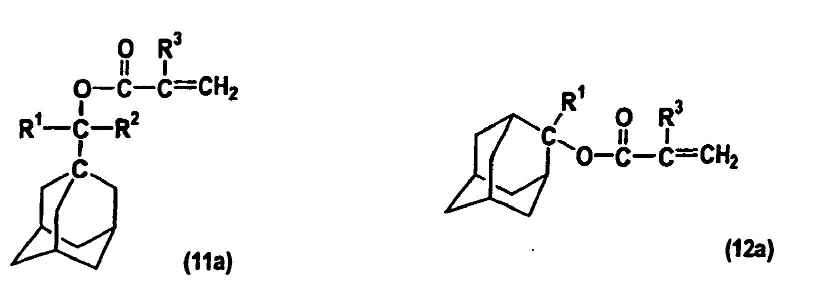 wherein R¹ and R² are the same or different and each represents a C₁₋₄alkyl group, and R³ represents a hydrogen atom or methyl group; and
a lactone ring-containing monomer unit.

2. A photoresist copolymer according to Claim 1, wherein the proportion of the monomers (11a) and (12a) in the copolymer is 15 to 90 % by weight.

3. A photoresist resin composition comprising a photoresist copolymer recited in Claim 1 and a photoactive acid precursor.

4. The photoresist resin composition according to Claim 3, which contains 0.1 to 30 parts by weight of the photoactive acid precursor relative to 100 parts by weight of the copolymer.

5. A method of forming a pattern which comprises the steps of:
forming a layer comprising the photoresist resin composition of Claim 3 on a substrate;
subjecting said layer to pattern exposure; and
developing the exposed coating layer to form a pattern.

## Revendications

1. Copolymère photorésistant comprenant
un motif monomère correspondant à un monomère représenté par la formule suivante (11a) ou (12a) : 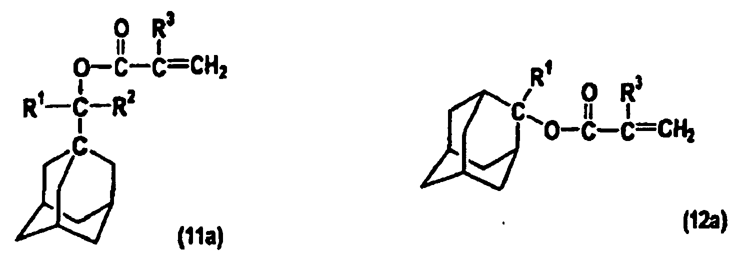 où R¹ et R² sont identiques ou différents et représentent chacun un groupe alkyle en C₁-C₄, et R³ représente un atome d'hydrogène ou un groupe méthyle ;
er
un motif monomère contenant un cycle lactone.

2. Copolymère photorésistant selon la revendication 1, dans lequel la proportion des monomères (11a) et (12a) dans le copolymère est de 15 à 90 % en poids.

3. Composition de résine photorésistante comprenant un copolymère photorésistant selon la revendication 1 et un précurseur acide photoréactif.

4. Composition de résine photorésistante selon la revendication 3, qui contient de 0,1 à 30 parties en poids de précurseur acide photoréactif par rapport à 100 parties en poids de copolymère.

5. Procédé de formation d'un motif qui comprend les étapes consistant à :
former une couche comprenant la composition de résine photorésistante selon la revendication 3 sur un substrat ;
soumettre ladite couche à une exposition de motif ; et
développer la couche protectrice exposée pour former un motif.

## Patentansprüche

1. Photoresistcopolymer, umfassed
eine Monomereinheit, die einem Monomer entspricht, dargestellt durch die folgende Formel (11a) oder (12a): 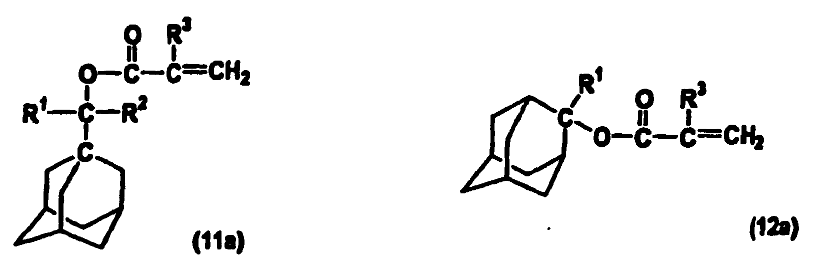 worin R¹ und R², die gleich oder verschieden sein können, jeweils eine C₁₋₄ Alkylgruppe bedeuten, und R³ ist ein Wasserstoffatom oder eine Methylgruppe; und
eine Monomereinheit mit einem Lactonring.

2. Photoresistcopolymer nach Anspruch 1, wobei der Anteil der Monomere (11 a) und (12a) in dem Copolymer im Bereich von 15 bis 90 Gew.% liegt.

3. Photoresistharzzusammensetzung, umfassend das Photoresistcopolymer nach Anspruch 1 und einen Vorläufer, der beim Belichten eine Säure bildet.

4. Photoresistharzzusammensetzung nach Anspruch 3, enthaltend 0,1 bis 30 Gewichtsteile des Vorläufers, der beim Belichten eine Säure bildet, bezogen auf 100 Gewichtsteile des Copolymers.

5. Verfahren zum Erzeugen eines Musters, umfassend die folgenden Schritte:
das Aufbringen einer Schicht mit der Photoresistharzzusammensetzung nach Anspruch 3 auf einem Substrat;
das musterförmige Belichten der Schicht; und
das Entwickeln der auf dem Substrat aufgebrachten belichteten Schicht, um ein Muster zu erzeugen.
